# FASCICULE DE BREVET EUROPEEN

(11) **EP 0 971 639 B1**
(45) Date de publication et mention de la délivrance du brevet: **22.01.2003**
(21) Numéro de dépôt: 98920564.6
(22) Date de dépôt: 03.04.1998
(51) Int. Cl.: A61B 17/70

(54) **DISPOSITIF D'OSTEOSYNTHESE DU RACHIS A FIXATION DE TIGE INTERVERTEBRALE DESAXEE**
VORRICHTUNG ZUR WIRBELSÄULEN-OSTEOSYTHESE MIT VERSETZT ANGEORDNETEM ZWISCHENWIRBELVERANKERUNGSSTAB
DEVICE FOR BACKBONE OSTEOSYNTHESIS WITH OFFSET INTERVERTEBRAL FIXING ROD

(30) Priorité: 04.04.1997 FR 9704133
(43) Date de publication de la demande: 19.01.2000
(73) Titulaire: Stryker France S.A., 93290 Tremblay-en-France (FR)
(72) Inventeur: LE COUEDIC, Régis, F-33160 Saint-Médard-en-Jalles (FR); BACCELLI, Christian, F-33650 Saint-Médard-d'Eyrans (FR); CONCHY, Frédéric, F-33000 Bordeaux (FR)
(74) Mandataire: Le Forestier, Eric
(86) Numéro de dépôt international: FR9800679
(87) Numéro de publication internationale: WO98044859

(56) Documents cités:
- WO-A-95/02372
- WO-A-96/29947
- DE-A- 19 512 709
- US-A- 5 002 542
- US-A- 5 575 791

## Description

La présente invention a trait d'une façon générale les dispositifs pour l'ostéosynthèse du rachis.

On connaît déjà en particulier par le document FR-A-2 659 546 un implant pour l'ostéosynthèse du rachis qui comprend une vis pédiculaire munie d'une tête en forme générale de diapason pouvant recevoir directement une tige cylindrique de liaison intervertébrale. Une bague fendue et un bouchon fileté de serrage opérant entre les deux branches de la tête permettent le blocage de l'ensemble, avec possibilité d'angulation entre la tige et l'axe de la vis pédiculaire.

La pose de ce type d'implant par le chirurgien ne présente pas de difficulté majeure dès lors que les têtes des différentes vis pédiculaires sont suffisamment bien alignées les unes par rapport aux autres, car dans ce cas la tige de liaison intervertébrale peut être relativement facilement placée dans lesdites têtes, sans sollicitations ou torsions excessives.

Toutefois, notamment selon les types de visées pédiculaires effectuées par les chirurgiens, et aussi selon l'orientation des pédicules propres à chaque patient, il se trouve fréquemment que les axes des vis pédiculaires présentent des inclinaisons importantes par rapport au plan sagittal, et il s'ensuit que les logements pour la tige de liaison intervertébrale, définis par les têtes des différentes vis, peuvent présenter des défauts d'alignement très marqués.

La solution qui consiste dans ce cas à déformer très significativement la tige pour la forcer à suivre le chemin imposé est soit impraticable, du fait des efforts de déformation considérables qui devraient être impartis, soit dangereuse, car elle risque de fragiliser la tige.

On connaît par ailleurs différents dispositifs destinés à autoriser la fixation et le blocage d'une tige de façon décalée latéralement par rapport à une vis pédiculaire.

Les documents DE-A-195 12 709, US-A-5 575, US-A-5 002 542, WO-A-95 02372 et WO-A-96 29947 donnent des exemples de tels dispositifs.

Le document US-A-5 575 791 divulgue un dispositif selon le préambule de la revendication 1.

Toutefois ces dispositifs connus présentent tous des inconvénients. Ainsi, certains présentent un encombrement important, notamment en hauteur, ce qui est tout à fait indésirable et diminue considérablement l'avantage des dispositifs à vis de type « Diapason » tels que décrits plus haut en termes de compacité. D'autres présentent une construction complexe, avec de nombreuses pièces. Certains sont par ailleurs incapables, de par leur structure, à recevoir des tiges à une distance convenable des pédicules, distance qui est imposée par ailleurs par les vis de type « Diapason ». Enfin certains ce ces dispositifs connus sont inaptes à effectuer un blocage suffisamment ferme et solide de la tige par rapport à l'ancrage osseux.

La présente invention a donc pour objet de pallier ces inconvénients et de proposer des moyens simples, et donc ne compliquant pas significativement les opérations de pose, permettant de relier rigidement un implant osseux de type classique à une tige de liaison intervertébrale alors que la tige est sensiblement désaxée par rapport à l'implant osseux.

Un autre objet de l'invention est de pouvoir utiliser, quelle que soit l'importance de ces décalages, un même type de vis pédiculaire dans toutes les vertèbres.

Un autre objet encore est de proposer une fixation désaxée d'une tige de liaison également au niveau d'une fixation sacrée d'un type particulier.

Ainsi la présente invention propose un dispositif d'ostéosynthèse du rachis selon la revendication 1.

Des aspects préférés, mais non limitatifs, du dispositif selon l'invention sont les suivants :
- la liaison à rotule comprend un siège généralement sphérique prévu dans une tête de l'élément d'ancrage osseux et une bague compressible essentiellement complémentaire reçue dans ledit siège, et ladite première partie de l'élément intermédiaire comprend un appendice cylindrique reçu dans ladite bague.
- ledit appendice cylindrique est de section circulaire.
- ledit appendice cylindrique présente une longueur telle qu'avant blocage, il peut coulisser par rapport à la bague pour faire varier l'entraxe entre l'élément d'ancrage osseux et la tige de liaison intervertébrale.
- ledit appendice cylindrique présente dans la région de son extrémité libre un élargissement destiné à éviter que ledit appendice ne s'échappe librement de la bague avant blocage.
- la bague est une bague fendue et en ce que l'élargissement présente un diamètre tel que l'appendice peut être engagé à force à travers la bague avec déformation élastique temporaire de cette dernière.
- ladite première partie de l'élément intermédiaire comprend un logement pour une tête de l'élément d'ancrage osseux, et la liaison à rotule comprend un siège généralement sphérique défini dans ledit logement et une paroi sphérique essentiellement complémentaire formée sur ladite tête.
- les moyens de blocage de la liaison à rotule comprennent un bouchon fileté vissé dans une ouverture dudit logement opposée à son siège.
- lesdites branches de la pince définissent entre elles un passage évidé apte à recevoir la tige de liaison intervertébrale, et en ce que ledit organe de serrage est constitué par une vis.

D'autres aspects, buts et avantages de la présente invention apparaîtront mieux à la lecture de la description détaillée suivante d'une forme de réalisation préférée de celle-ci, donnée à titre d'exemple et faite en référence au dessin annexé, sur lequel :
la figure 1 est une vue en coupe transversale éclatée d'un dispositif d'ostéosynthèse selon une première forme de réalisation de l'invention,
la figure 2 est une vue en coupe transversale après assemblage partielle du dispositif de la figure 1,
les figures 3 et 4 sont des vues en coupe transversale du dispositif des figures 1 et 2 dans deux positions de blocage différentes, certains organes du dispositif n'étant pas illustrés par souci de simplification,
la figure 5 est une vue de profil d'une pièce d'un dispositif d'ostéosynthèse selon une deuxième forme de réalisation de l'invention,
la figure 6 est une vue de face, selon la flèche VI de la figure 5,
la figure 7 est une vue de dos, selon la flèche VII de la figure 5,
la figure 8 est une vue de dessus, selon la flèche VIII de la figure 5,
la figure 9 est une vue en perspective de l'élément des figures 5 à 8,
la figure 10 est une vue en perspective du dispositif d'ostéosynthèse selon la deuxième forme de réalisation de l'invention à l'état assemblé, et
la figure 11 est une vue en coupe transversale du dispositif de la figure 10.

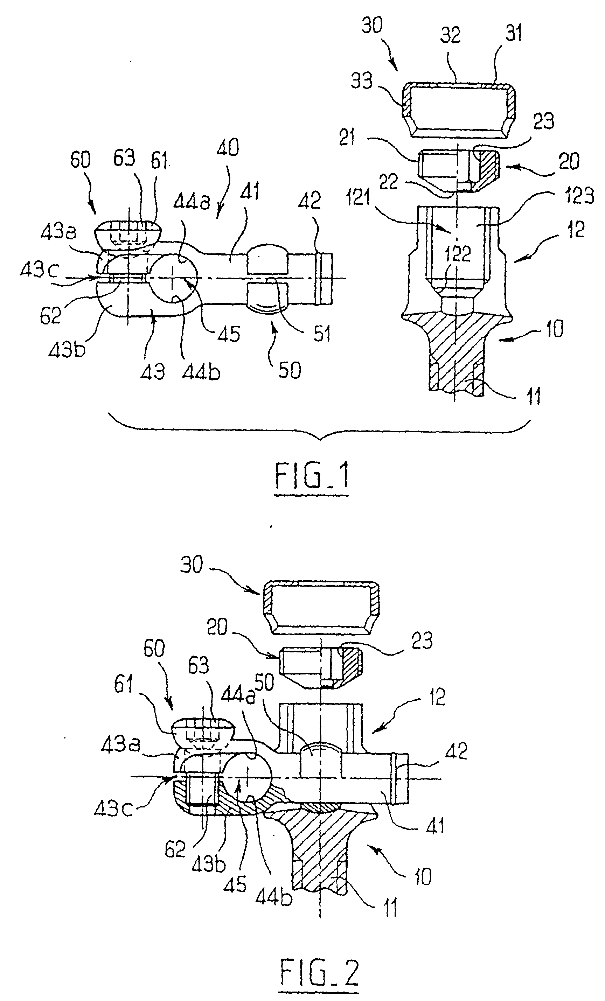

En référence au dessin, et tout d'abord aux figures 1 à 4, on a représenté une première forme d'un dispositif d'ostéosynthèse pour le rachis, qui comprend un élément d'ancrage osseux 10 en l'espèce sous la forme d'une vis pédiculaire, avec une partie filetée 11 destinée à coopérer avec l'os et une tête 12.

Cette tête 12 comporte deux branches 123 en forme générale de diapason, filetées intérieurement et définissant une cavité 121 au fond de laquelle est formé un siège sphérique 122.

Un bouchon fileté 20 possédant un filetage externe 21 et une portée 22 généralement plane ou concave est apte à être vissé entre les deux branches à l'aide d'un outil introduit dans une empreinte de vissage 23, comme expliqué plus loin.

Enfin un capuchon 30, possédant une face supérieure 31 dans laquelle est formée une ouverture 32 pour l'outil de vissage précité et une jupe périphérique descendante 33, peut être appliqué sur le dessus de la tête 12 en recouvrant les branches 123, afin notamment d'éviter l'écartement desdites branches lors du vissage précité.

Le dispositif selon cette première forme de réalisation comprend par ailleurs un élément intermédiaire 40 dont une première extrémité, à droite sur la figure 1, définit un appendice cylindrique de révolution 41, appelé tige par la suite. Sur cette tige 41 est placée une bague 50 possédant un contour extérieur généralement sphérique et un passage traversant cylindrique d'un diamètre légèrement supérieur à celui de la tige 41. La bague est fendue en 51 pour lui conférer un certain degré de déformabilité élastique, comme on le verra plus loin.

La tige 41 possède au voisinage de son extrémité libre une nervure peu saillante 42 qui permet d'éviter que la bague 50 ne puisse s'échapper librement de la tige 41 une fois qu'elle a été placée sur celle-ci en franchissant à force ladite nervure.

L'élément intermédiaire 40 possède, à son extrémité opposée, une partie formant pince 43 possédant deux branches 43a, 43b disposées l'une au-dessus de l'autre. Dans la région de leur racine, les deux branches possèdent chacune un évidement essentiellement semi-cylindrique, respectivement 44a, 44b, ces deux évidements définissant conjointement un passage cylindrique 45 orienté perpendiculairement à l'axe de la tige 41.

Entre lesdits évidements et leurs extrémités libres, les branches 43a, 43b sont séparées par un interstice 43c et sont traversées par deux passages cylindriques coaxiaux, orientés perpendiculairement au passage 45 et à la tige 41. Le passage supérieur est lisse, tandis que le passage inférieur et taraudé.

Une vis de serrage 60 est prévu pour être introduite et vissée dans ces passages, cette vis comportant de façon classique en soi une tige filetée 62, une tête plus large 61 et une empreinte 63 pour outil de serrage, formée dans la tête 61.

On observera ici que l'ensemble constitué de la vis pédiculaire 10, du bouchon fileté 20, du capuchon 30 et de la bague 50 est conforme à la description du document FR-A-2 659 546.

La figure 2 illustre le dispositif de la figure 1 dans un état partiellement monté.

Ainsi, la vis pédiculaire 10 ayant été préalablement ancrée dans une vertèbre, la tige 41 munie de la bague 50 a été mise en plae dans l'espace intérieur 121 de la tête 12 de la vis 10, la bague 50 s'appuyant au niveau de sa région inférieure sur la portée sphérique concave 122 ménagée au fond dudit espace intérieur.

Une fois que l'élément intermédiaire 40 occupe la position qui convient, le bouchon 20 est prémonté dans la tête 12, le capuchon est mis en place au sommet de cette dernière, et le serrage du bouchon 20 est effectué à l'aide d'un outil.

Le bouchon 20 comprime et déforme alors la bague 50, pour ainsi bloquer la tige 41 dans la position voulue tant en translation le long de son axe qu'angulairement.

Le passage 45 est destiné quant à lui à recevoir une tige de liaison intervertébrale (non représentée), reliée à d'autres éléments d'ancrage osseux identiques ou différents.

Le serrage de la vis 60 permet d'assurer, par pincement entre les branches 43a, 43b, le blocage de la tige de liaison précitée.

Ainsi le dispositif décrit ci-dessus permet, par des moyens simples à fabriquer et à manipuler, de fixer une tige de liaison intervertébrale de façon désaxée et éventuellement décalée en hauteur, par rapport à un implant de type classique tel que décrit notamment dans FR-A-2 659 546, avec des possibilités d'ajustement très grandes obtenues d'une part en choisissant la position de la bague 50 sur la tige 41 avant blocage à l'aide du bouchon 20 et d'autre part en choisissant l'orientation angulaire de cette même tige 41, également avant blocage à l'aide du bouchon 20.

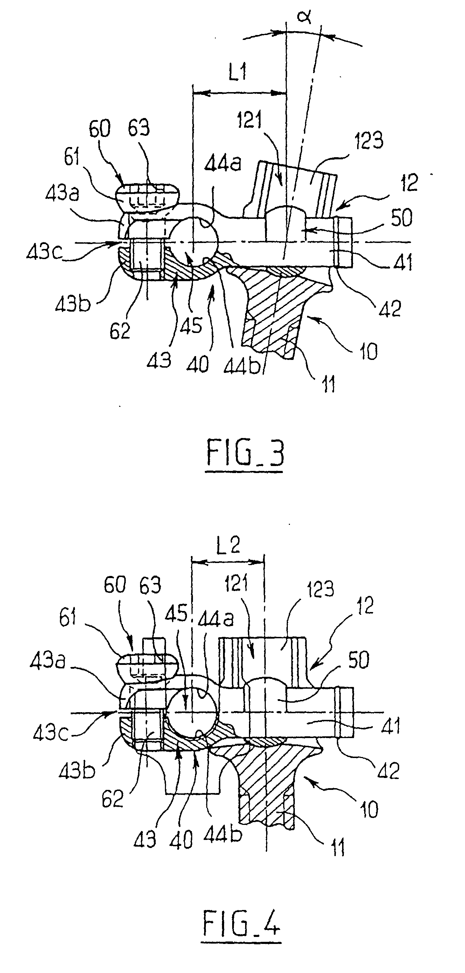

Ainsi la figure 3 des dessins illustre le cas où l'entraxe entre la vis pédiculaire 10 et la tige de liaison présente une certaine valeur L1, tandis que l'angle □ entre l'axe de la vis pédiculaire 10 et le plan perpendiculaire à l'axe de la tige 41 est non nul.

Dans le cas de la figure 4, l'entraxe précité a été réduit à une valeur L2 inférieure à L1, tandis que l'axe de la vis pédiculaire 10 est situé dans le plan perpendiculaire à l'axe de la tige 41 (□ = 0□).

Par ailleurs, la position de l'élément 40 par rapport à la tige de liaison intervertébrale peut être si nécessaire ajustée avant le blocage à l'aide de la vis 60.

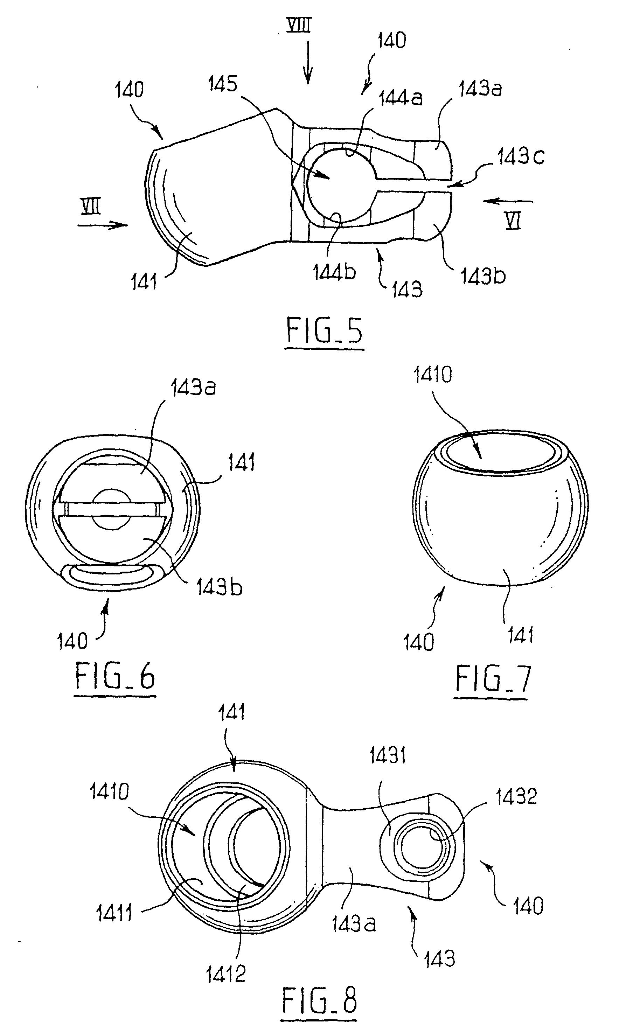

En référence maintenant aux figures 6 à 11, on a illustré un dispositif d'ostéosynthèse pour colonne vertébrale selon une seconde forme de réalisation de l'invention. Sur ces figures, des éléments ou parties identiques ou similaires à ceux de la forme de réalisation précédente sont désignés dans la mesure du possible par les mêmes signes de référence.

Ce dispositif comprend une vis 10, plus particulièrement destinée à un ancrage dans le sacrum, cette vis comprenant une tige filetée 11 surmontée par une tête sphérique 13 dans laquelle est formée, du côté opposée à la tige filetée 11, une empreinte en creux 131 pour le vissage.

Le dispositif comprend un élément intermediaire 140 dont une partie 141 présente la forme d'un corps généralement sphérique creux 141. La cavité 1410 définie dans ce corps comporte une partie cylindrique taraudée 1411 et, au-dessous de ce taraudage, un siège sphérique concave 1412 de même diamètre que celui de la tête sphérique 13 de la vis de fixation sacrée 10.

Cette cavité 1410 débouche vers le haut au niveau du taraudage et vers le bas par un orifice situé au fond du siège sphérique et dont la taille est sensiblement supérieure à la section de la tige filetée 11 de la vis 10.

A l'intérieur de la cavité 1410 peut être engagé à partir du haut, par vissage, un bouchon 20 possédant un filetage externe 21 apte à coopérer avec le taraudage 1411 et, sur sa face supérieure, une empreinte traversante 23 pour le serrage à l'aide d'un outil. La face inférieure du bouchon 20 définit, autour du débouché de l'empreinte 23, une surface sphérique en creux 24 de même diamètre que celui de la tête sphérique 13 de la vis 10.

L'élément 140 possède par ailleurs, en saillie latéralement à partir du corps 141, un prolongement formant pince 143 analogue à la pince 43 des figures 1 à 4.

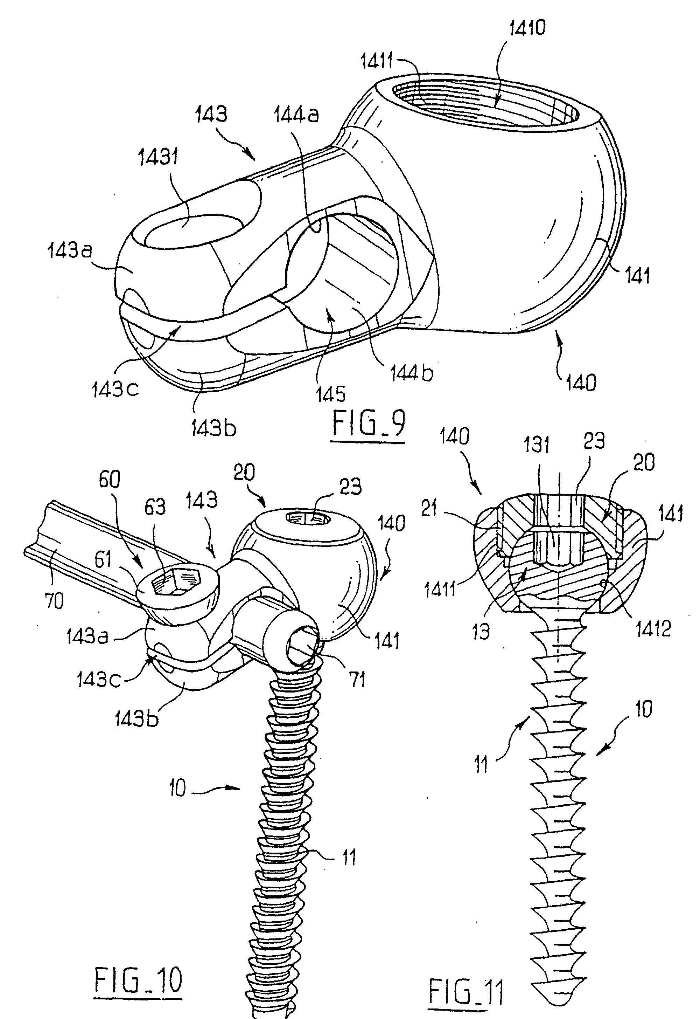

Plus précisément, deux branches 143a, 143b s'étendent le long l'une de l'autre, définissent au voisinage de leur racine deux renfoncements essentiellement semi-cylindriques 144a, 144b définissant un passage traversant 145 pour une tige de liaison intervertébrale 70, illustrée partiellement sur la figure 10.

Entre ce passage et leurs extrémités libres, les branches 143a, 143b sont séparées par un interstice 143c et traversées respectivement par un alésage lisse 1431 et par un alésage taraudé 1432 pour recevoir une vis de serrage 60 identique à celle des figures 1 à 4.

Le montage du dispositif s'effectue :
- en introduisant la vis de fixation sacrée 10 dans la cavité 1410 du corps 141 de la pièce 140 de telle sorte que sa tête 13 termine sa course dans ladite cavité, la partie filetée 11 débordant alors au-dessous du corps 141 et pouvant être vissée dans le sacrum à l'aide d'un outil engagé dans l'empreinte 131;
- en vissant le bouchon 20 dans la cavité 141 au-dessus de la tête 13, sans le serrer, en donnant alors à la pince 143, recevant la tige de liaison 70, l'inclinaison souhaitée par rapport à l'axe de la vis 10, et en effectuant alors le serrage, à l'aide d'un outil engagé dans l'empreinte 23; la tête de vis 13 est alors fermement bloquée par rapport à l'élément 140 dans la position souhaitée;
- en bloquant la tige de liaison 70 dans son passage 145 à l'aide de la vis 60 (cette opération pouvant être aussi effectuée préalablement à au moins l'une des étapes précédentes).

Ainsi cette forme de réalisation permet, à l'aide d'une pièce unique 140, de réaliser une liaison excentrée entre une vis de fixation sacrée et une tige de liaison intervertébrale, reliée par d'autres moyens, classiques en eux-mêmes, à des vertèbres adjacentes au sacrum.

On observera ici que la forme généralement arrondie des contours de la pièce 140 (ainsi que de la partie formant pince 43 dans la forme de réalisation précédente) permet de minimiser les lésions des tissus adjacents.

Bien entendu, les différents composants des deux dispositifs d'ostéosynthèse décrits ci-dessus sont réalisés en un matériau biocompatible tel que l'acier inoxydable ou un alliage de titane.

## Revendications

1. Dispositif d'ostéosynthèse du rachis, comprenant :
un élément d'ancrage osseux (10),
une tige de liaison intervertébrale (70) apte à relier ledit élément d'ancrage osseux avec d'autres éléments d'ancrage,
un élément intermédiaire (40, 140),
une liaison à rotule (122, 50; 1412, 13) entre une première partie (41; 141) de l'élément intermédiaire et l'élément d'ancrage osseux (10),
une liaison par serrage (43 ; 143) entre une seconde partie de l'élément intermédiaire et la tige de liaison intervertébrale, et
des moyens (20, 60) de blocage de la liaison à rotule et de la liaison par serrage,
dispositif **caractérisé en ce que** ladite liaison par serrage est constituée par une pince (43; 143) comportant deux branches (43a, 43b; 143a, 143b) orientées latéralement à partir de la liaison à rotule, ouvertes à l'opposé de celle-ci et emprisonnant entre elles la tige de liaison intervertébrale, et **en ce qu'**il est prévu des premiers moyens de blocage agissant sur la liaison à rotule et des seconds moyens de blocage agissant sur la pince dans la région des extrémités desdites branches situées, par rapport à la tige, du côté opposé à la liaison à rotule, pour solliciter lesdits branches l'une vers l'autre.

2. Dispositif selon la revendication 1, **caractérisé en ce que** la liaison à rotule comprend un siège généralement sphérique (122) prévu dans une tête (12) de l'élément d'ancrage osseux et une bague compressible essentiellement complémentaire (50) reçue dans ledit siège, **en ce que** ladite première partie de l'élément intermédiaire comprend un appendice cylindrique (41) reçu dans ladite bague (50).

3. Dispositif selon la revendication 2, **caractérisé en ce que** ledit appendice cylindrique (41) est de section circulaire.

4. Dispositif selon l'une des revendications 2 et 3, **caractérisé en ce que** ledit appendice cylindrique (41) présente une longueur telle qu'avant blocage, il peut coulisser par rapport à la bague (50) pour faire varier l'entraxe entre l'élément d'ancrage osseux (10) et la tige de liaison intervertébrale (70).

5. Dispositif selon la revendication 4, **caractérisé en ce que** ledit appendice cylindrique (41) présente dans la région de son extrémité libre un élargissement (42) destiné à éviter que ledit appendice ne s'échappe librement de l'a bague (50) avant blocage.

6. Dispositif selon la revendication 5, **caractérisé en ce que** la bague (50) est une bague fendue et **en ce que** l'élargissement (42) présente un diamètre tel que l'appendice peut être engagé à force à travers la bague avec déformation élastique temporaire de cette dernière.

7. Dispositif selon la revendication 1, **caractérisé en ce que** ladite première partie de l'élément intermédiaire comprend un logement (141) pour une tête (13) de l'élément d'ancrage osseux, et **en ce que** la liaison à rotule comprend un siège généralement sphérique (1412) défini dans ledit logement et une paroi sphérique essentiellement complémentaire formée sur ladite tête (13).

8. Dispositif selon la revendication 7, **caractérisé en ce que** les moyens de blocage de la liaison à rotule comprennent un bouchon fileté (20) vissé dans une ouverture dudit logement opposée à son siège (1412).

9. Dispositif selon l'une des revendications 1 à 8, **caractérisé en ce que** lesdites branches (43a, 43b; 143a, 143b) de la pince définissent entre elles un passage évidé (45; 145) apte à recevoir la tige de liaison intervertébrale (70), et **en ce que** ledit organe de serrage est constitué par une vis (60).

## Patentansprüche

1. Vorrichtung für die Osteosynthese der Wirbelsäule , die folgendes aufweist:
ein Element für die Knochenverankerung (10),
eine Stange für die Verbindung der Wirbel (70), mit deren Hilfe das Element für die Knochenverankerung mit anderen Verankerungselementen verbunden werden kann,
ein Zwischenelement (40,140),
ein Kugelgelenk (122, 50; 1412, 1413), welches zwischen einem ersten Teil (41; 141) des Zwischenelementes und dem Element für die Knochenverankerung (10) angeordnet ist,
ein Spannelement (43, 143), welches zwischen einem zweiten Teil des Zwischenelementes und der Stange für die Verbindung der Wirbel angeordnet ist, und
Mittel (20, 60) für die Blockierung des Kugelgelenkes und dem Spannelement,
**dadurch gekennzeichnet, dass**
das Spannelement aus einer Klammer (43; 143) mit zwei Schenkeln (43a, 43b; 143a, 143b) besteht, die seitlich von dem Kugelgelenk abstehen und gegenüber diesem offen sind und zwischen diesen Schenkeln die Stange für die Verbindung der Wirbel hält, und dadurch, dass erste Blockiermittel vorgesehen sind, die auf das Kugelgelenk einwirken, sowie zweite Blockiermittel, welche auf die Klammer im Bereich der Enden der Schenkel einwirken, welche gegenüber der Stange auf der gegenüberliegenden Seite des Kugelgelenkes liegen, um die beiden Schenkel zu schließen.

2. Vorrichtung nach Anspruch 1,
**dadurch gekennzeichnet, dass**
das Kugelgelenk einen generell kugelförmigen Sitz (122) aufweist, der in einen Kopf (12) des Elementes für die Knochenverankerung eingebracht ist, sowie einen weitgehend komplementären komprimierbaren Ring (50), welcher in diesen Sitz eingesetzt ist, und dadurch, dass der erste Teil des Zwischenelementes einen zylindrischen Ansatz (41) aufweist, der in diesen Ring (50) eingesetzt ist.

3. Vorrichtung nach Anspruch 2,
**dadurch gekennzeichnet, dass**
der zylindrische Ansatz (41) einen kreisförmigen Querschnitt hat.

4. Vorrichtung nach einem der Ansprüche 2 und 3,
**dadurch gekennzeichnet, dass**
der zylindrische Ansatz (41) eine Länge hat, die so bemessen ist, dass er vor der Blockierung gegenüber dem Ring (50) gleiten kann, um dadurch den Mittenabstand zwischen dem Element für die Knochenverankerung (10) und der Stange für die Verbindung der Wirbel (70) zu variieren.

5. Vorrichtung nach Anspruch 4,
**dadurch gekennzeichnet, dass**
der zylindrische Ansatz (41) im Bereich seines freien Endes eine Erweiterung (42) besitzt, die verhindert, dass dieser Ansatz vor der Blockierung ungehindert aus dem Ring (50) entweichen kann.

6. Vorrichtung nach Anspruch 5,
**dadurch gekennzeichnet, dass**
der Ring (50) ein Schlitzring ist, und dass die Erweiterung (42) einen Durchmesser hat, der so bemessen ist, dass der Ansatz unter Druck über den Ring unter vorübergehender elastischer Verformung des Letzteren in Eingriff treten kann.

7. Vorrichtung nach Anspruch 1,
**dadurch gekennzeichnet, dass**,
der erste Teil des Zwischenelementes einen Lagersitz (141) für einen Kopf (13) des Elementes für die Knochenverankerung aufweist, und dadurch, dass das Kugelgelenk einen generell kugelförmigen Sitz (1412) aufweist, der in diesen Lagersitz eingebracht ist, sowie eine weitgehend komplementäre kugelförmige Wand, welche an dem Kopf (13) ausgebildet ist.

8. Vorrichtung nach Anspruch 7,
**dadurch gekennzeichnet, dass**
die Mittel für die Blockierung des Kugelgelenkes einen Schraubdeckel (20) aufweisen, der in eine Öffnung dieses Lagersitzes gegenüber dem Sitz (1412) eingeschraubt werden kann.

9. Vorrichtung nach einem der Ansprüche 1 bis 8,
**dadurch gekennzeichnet, dass**
die Schenkel (43a, 43b; 143a, 143b) der Klammer untereinander einen erweiterten Durchgang (45; 145) bilden, in den die Stange für die Verbindung der Wirbel (70) eingesetzt werden kann, und dadurch, dass die Spannvorrichtung aus einer Schraube (60) besteht.

## Claims

1. An osteosynthesis device for the spine, the device comprising:
a bone anchor element (10),
an intervertebral link rod (70) suitable for connecting said bone anchor element to other anchor elements,
an intermediate element (40; 140),
a ball-and-socket link (122, 50; 1412, 13) between a first portion (41; 141) of the intermediate element and the bone anchor element (10),
a clamping link (43; 143) between a second portion of the intermediate element and the intervertebral link rod, and
locking means (20, 60) for locking the ball-and-socket link and the clamping link,
which device is **characterized in that** said clamping link is constituted by a clamp (43; 143) having two branches (43a, 43b; 143a, 143b) extending laterally from the ball-and-socket link, open away therefrom, and holding the intervertebral link rod captive therebetween, and **in that** first locking means are provided acting on the clamp in the end region of said branches situated, relative to the rod, on the opposite side to the ball-and-socket link, so as to urge said branches towards each other.

2. A device according to claim 1, **characterized in that** the ball-and-socket link comprises a generally spherical seat (122) provided in a head (12) of the bone anchor element and an essentially complementary compressible ring (50) received in said seat, and **in that** said first portion of the intermediate element comprises a cylindrical appendix (41) received in said ring (50).

3. A device according to claim 2, **characterized in that** said cylindrical appendix (41) is of circular section.

4. A device according to claim 2 or 3, **characterized in that** said cylindrical appendix (41) is of a length such that prior to locking, it is capable of sliding relative to the ring (50) to vary the axial offset between the bone anchor element (10) and the intervertebral link rod (70).

5. A device according to claim 4, **characterized in that** said cylindrical appendix (41), in the region of its free end, presents an enlargement (42) for preventing said appendix from escaping freely from the ring (50) prior to locking.

6. A device according to claim 5, **characterized in that** the ring (50) is a split ring and **in that** the enlargement (42) has a diameter such that the appendix can be engaged by force through the ring with temporary elastic deformation thereof.

7. A device according to claim 1, **characterized in that** said first portion of the intermediate element has a housing (141) for a head (13) of a bone anchor element, and **in that** the ball-and-socket link has a generally spherical seat (1412) defined in said housing and an essential complementary spherical wall formed on said head (13).

8. A device according to claim 7, **characterized in that** the means for locking the ball-and-socket link comprise a threaded plug (20) screwed into an opening of said housing remote from its seat (1412).

9. A device according to any one of claims 1 to 8, **characterized in that** said branches (43a, 43b; 143a, 143b) of the clamp define an empty passage (45; 145) between them suitable for receiving the intervertebral link rod (70), and **in that** said clamping member is constituted by a screw (60).
